# EUROPÄISCHE PATENTSCHRIFT

(11) **EP 4 144 383 B1**
(45) Veröffentlichungstag und Bekanntmachung des Hinweises auf die Patenterteilung: **17.07.2024**
(21) Anmeldenummer: 21194248.7
(22) Anmeldetag: 01.09.2021
(51) Int. Cl.: A61L 9/12, B60H 3/00, A61L 9/04

(54) **BEDUFTUNGSVORRICHTUNG FÜR EIN FAHRZEUG**
AIR FRESHENER DEVICE FOR A VEHICLE
DISPOSITIF DE DIFFUSION DE PARFUM DANS UN VÉHICULE

(43) Veröffentlichungstag der Anmeldung: 08.03.2023
(73) Patentinhaber: Supair-Tel AG, 8152 Glattbrugg (CH)
(72) Erfinder: Guggenheim, Rudolf Heinrich, 8832 Wollerau (CH)
(74) Vertreter: E. Blum & Co. AG

(56) Entgegenhaltungen:
- DE-A1- 102008 031 156
- DE-A1- 102011 001 637
- GB-A- 2 542 439

## Beschreibung

### Gebiet der Erfindung

Die Erfindung betrifft eine Beduftungsvorrichtung für ein Fahrzeug mit einem Gehäuse und einem im Gehäuse angeordneten Duftstoffbehälter. Das Gehäuse umfasst eine Haltevorrichtung zum Anbringen der Beduftungsvorrichtung an einer Lüftung im Innenraum eines Fahrzeuges. Im Weiteren umfasst das Gehäuse eine Einlassseite und eine Auslassseite. An der Einlassseite ist eine Einlassöffnung zum Einströmen von Luft in das Gehäuse angeordnet und an der Auslassseite ist eine Auslassöffnung zum Ausströmen von Luft aus dem Gehäuse angeordnet. Die Auslassseite weist eine längliche Form mit einer Längsseite und einer Kurzseite auf.

### Hintergrund

Es sind Beduftungsvorrichtungen bekannt, welche im Fahrzeuginneren aufgehängt werden können und z.B. ein mit Duftstoff getränktes Material aufweisen. Solche Vorrichtungen sind kostengünstig herstellbar, ihre Beduftungswirkung kann aber ungleichmässig sein und der in der Regel am Innenrückspiegel aufgehängte Duftkörper kann sichtbehindernd sein.

Im Weiteren sind Behälter mit Duftstoff bekannt, welche an einem Lüftungsgitter im Innenraum eines Fahrzeuges befestigt werden. Der Behälter für den Duftstoff weist Einlassöffnungen auf, sodass Luft aus der Lüftung in den Behälter hineinströmt, mit dem Duftstoff in Kontakt gerät und angereichert mit Duftmolekülen den Behälter durch Auslassöffnungen verlässt. Solche Behälter können mittels eines Klemmteils am Lüftungsgitter befestigt und von diesem auch wieder entfernt werden.

DE 10 2011 001637 A1 zeigt eine Beduftungsvorrichtung für ein Fahrzeug mit einem Behälter für einen Duftstoff. Die Beduftungsvorrichtung kann an einem Lüftungsschlitz im Innenraum des Fahrzeuges angebracht werden. Ein Verschieben des Duftstoffbehälters ist nicht vorgesehen.

GB 2 542 439 A zeigt eine Beduftungsvorrichtung mit einem verschiebbaren Duftstoffbehälter.

DE 10 2008 031156 A1 zeigt eine Beduftungsvorrichtung mit einer Duftstoffpatrone.

### Darstellung der Erfindung

Es stellt sich die Aufgabe, eine Beduftungsvorrichtung bereitzustellen, welche einfach bedienbar ist.

Diese Aufgabe wird durch eine Beduftungsvorrichtung mit den Merkmalen des Anspruchs 1 gelöst.

Die erfindungsgemässe Beduftungsvorrichtung umfasst ein Gehäuse, in welchem ein Duftstoffbehälter angeordnet ist. Das Gehäuse umfasst
- eine Haltevorrichtung zum Anbringen der Beduftungsvorrichtung an einer Lüftung im Innenraum eines Fahrzeuges. Der Begriff "Fahrzeug" umfasst Landfahrzeuge, wie Personenkraftwagen und Lastkraftwagen, und auch Luftfahrzeuge.
- eine Einlassseite, an welcher eine Einlassöffnung zum Einströmen von Luft in das Gehäuse angeordnet ist. Ist die Beduftungsvorrichtung an der Lüftung des Fahrzeuges befestigt, so ist die Einlassseite insbesondere zur Lüftung hin gerichtet.
- eine, insbesondere der Einlassseite gegenüberliegende, Auslassseite, an welcher eine Auslassöffnung zum Ausströmen von Luft aus dem Gehäuse angeordnet ist. Ist die Beduftungsvorrichtung an der Lüftung des Fahrzeuges befestigt, so ist die Auslassseite insbesondere von der Lüftung abgewandt.

Insbesondere weist die Auslassseite eine längliche Form mit einer Längsseite und einer Kurzseite auf. Beispielsweise kann die Auslassseite eine Rechteckform aufweisen. Die Rechteckform weist eine Längsseite und eine Kurzseite auf. Ist das Rechteck beispielsweise ein Quadrat, so sind Längsseite und Kurzseite gleich lang. D.h. insbesondere können Längsseite und Kurzseite unterschiedlich lang oder gleich lang sein.

Die Beduftungsvorrichtung umfasst eine Schiebevorrichtung mit einem im Gehäuse angeordneten Schiebebehälter. Im Schiebebehälter ist der Duftstoffbehälter angeordnet. Mittels der Schiebevorrichtung können die Auslass- und/oder Einlassöffnung mittels einer Schiebebewegung zumindest teilweise, insbesondere vollständig, verschlossen werden. Die Beduftungsvorrichtung lässt sich somit in einfacher Weise öffnen und schliessen. Sie ist deshalb sehr einfach bedienbar.

Die Schiebevorrichtung ist derart ausgestaltet, dass sich bei der Schiebebewegung der Duftstoffbehälter relativ zum Gehäuse verschiebt. Dies hat den Vorteil, dass die Schiebebewegung fehlerfrei durchgeführt werden kann. Zwischen dem Duftstoffbehälter und der Schiebevorrichtung gibt es keine Relativbewegung, bei welcher ein Verhacken oder eine sonstige Blockade eintreten könnte.

Insbesondere ist der Duftstoffbehälter im Schiebebehälter derart angeordnet, dass sich der Duftstoffbehälter bei der Schiebebewegung nicht entlang des Gehäuses reibt. Somit können bei der Bewegung auch zwischen Duftstoffbehälter und Gehäuse keine Blockaden auftreten. Die Schiebebewegung erfolgt kontrolliert innerhalb des Gehäuses.

Mit Vorteil umfasst das Gehäuse Stege, auf welchen der Schiebebehälter abgestützt ist, oder welche der Schiebebehälter berührt. Zwischen den Stegen und dem Schiebebehälter entsteht dadurch eine Relativbewegung. Aufgrund der geringen Berührungsfläche zwischen Steg und Schiebebehälter wird die Reibung reduziert und der Schiebebehälter kann innerhalb des Gehäuses kontrolliert bewegt werden.

Insbesondere weist die Schiebevorrichtung einen Griff auf, insbesondere einen plattenförmigen Griff. Durch Betätigung des Griffs kann die Schiebevorrichtung verschoben werden, wobei der Griff weder durch die Auslassseite noch durch die Einlassseite ragt. Damit kann die Beduftungsvorrichtung eine kompakte Geometrie aufweisen, weil die Einlassseite und Auslassseite möglichst vollständig für Einlass- und Auslassöffnungen zur Verfügung stehen.

Insbesondere ist die Auslassöffnung in Form eines länglichen Schlitzes ausgestaltet. Insbesondere können an der Auslassseite mehrere Schlitze nebeneinander angeordnet sein. Der längliche Schlitz ist schräg zur Längsseite angeordnet. D.h. der Schlitz verläuft nicht parallel zur Längsseite. Insbesondere steht der längliche Schlitz in einem Winkel von mindestens 10°, insbesondere mindestens 20°, insbesondere mindestens 30°, zur Längsseite.

Im Weiteren kann der längliche Schlitz auch schräg zur Kurzseite angeordnet sind. Insbesondere in einem Winkel von mindestens 10°, insbesondere mindestens 20°, insbesondere mindestens 30°, zur Kurzseite. Dies bedeutet, dass der längliche Schlitz weder parallel zur Längsseite noch parallel zur Kurzseite angeordnet ist. Stehen Kurzseite und Längsseite zueinander in einem Winkel von 90°, so kann der längliche Schlitz z.B. in einem Winkel von 45° zur Kurzseite und in einem Winkel von 45° zur Längsseite angeordnet sein.

Wären die länglichen Schlitze parallel zur Kurzseite angeordnet, so könnten sich die Schlitze maximal über die Länge der Kurzseite erstrecken. Die Schlitze wären verhältnismässig kurz und es müssten umso mehr Schlitze auf der Auslassseite angeordnet werden, damit ein bestimmtes Luftvolumen den Behälter durchströmen könnte. Da zwischen zwei Schlitzen jeweils aus konstruktiven Gründen ein Mindestabstand erforderlich ist, müsste die Auslassseite entsprechend gross dimensioniert werden. Sind die Schlitze hingegen schräg zur Kurzseite angeordnet, kann das beschriebene Problem verhindert werden. Die Schlitze können mit einer grösseren Länge dimensioniert werden.

Wären die länglichen Schlitze parallel zur Längsseite angeordnet, so wäre eine Schiebevorrichtung, mit welcher die Schlitze geschlossen und geöffnet bzw. verdeckt und freigelegt werden können, konstruktiv schwierig zu bewerkstelligen. Eine Schiebebewegung in Richtung der Kurzseite ist konstruktiv schwierig und eine Schiebebewegung in Richtung der Längsseite würde eine Unterbrechung der Schlitze erfordern.

Aus konstruktiven Gründen ist es deshalb vorteilhaft, die schlitzförmigen Auslassöffnungen schräg zur Längsseite und/oder zur Kurzseite anzuordnen.

Vorteilhaft umfasst die Beduftungsvorrichtung drei längliche Schlitze, insbesondere wobei die drei länglichen Schlitze auf der Auslassseite angeordnet sind. Die Beduftungsvorrichtung kann auch weitere Schlitze aufweisen.

Mit Vorteil sind die drei länglichen Schlitze parallel zueinander und/oder sie sind äquidistant zueinander angeordnet. Mit Vorteil sind die drei länglichen Schlitze gleich lang. Insbesondere erstrecken sich die länglichen Schlitze im Wesentlichen über die gesamte Kurzseite der Auslassseite. Eine derartige Anordnung der länglichen Schlitze hat den Vorteil, dass diese möglichst kompakt auf der Auslassseite angeordnet werden können.

Insbesondere ist der Duftstoffbehälter der Beduftungsvorrichtung entnehmbar. Er kann in Form eines Stifts ausgestaltet sein. Das Gehäuse kann eine Gehäuseöffnung aufweisen, durch welche der Duftstoffbehälter in das Gehäuse einsetzbar und diesem wieder entnehmbar sein soll. Ein entnehmbarer Duftstoffbehälter hat den Vorteil, dass dieser nach Verbrauch ausgetauscht werden kann, aber das Gehäuse der Beduftungsvorrichtung weiter verwendet werden kann.

Insbesondere weist das Gehäuse einen Gehäusedeckel auf, mit welchem die Gehäuseöffnung geöffnet und geschlossen werden kann. Der Gehäusedeckel ist mittels eines Scharniers am Gehäuse befestigt. Mit dem Scharnier lässt sich der Gehäusedeckel in einfacher Weise mittels einer Drehbewegung öffnen und schliessen. Der Gehäusedeckel bleibt am Gehäuse befestigt und kann während dem Ersetzen des Duftstoffbehälters nicht verloren gehen.

Mit Vorteil ist genau eine einzige Gehäuseöffnung vorhanden. Nur durch diese kann der Duftstoffbehälter in das Gehäuse eingesetzt werden. Ist der Gehäusedeckel geschlossen, kann der Gehäusedeckel insbesondere eingerastet sein, damit sich der Gehäusedeckel bei Verwendung nicht ungewollt öffnet.

Vorteilhaft erstreckt sich der Gehäusedeckel entlang eines Abschnitts der Längsseite. Dies hat den Vorteil, dass die Gehäuseöffnung verhältnismässig gross ist und der Duftstoffbehälter komfortabel in das Gehäuse eingesetzt werden kann.

Im Weiteren kann die Beduftungsvorrichtung derart ausgestaltet sein, dass der Gehäusedeckel nur schliessbar ist, wenn der Duftstoffbehälter in korrekter Position und/oder korrekt ausgerichtet im Gehäuse eingesetzt ist, wobei es nur genau eine korrekte Position und/oder nur genau eine korrekte Ausrichtung gibt. Insbesondere weist der Duftstoffbehälter hierfür eine Vertiefung auf, in welche ein Vorsprung des Gehäuses oder der Schiebevorrichtung eingreift. Greifen Vertiefung und Vorsprung nicht ineinander, kann der Duftstoffbehälter nicht vollständig in das Gehäuse eingesetzt werden und der Gehäusedeckel lässt sich nicht schliessen. Der Benutzer bemerkt die Fehlplatzierung und korrigiert diese.

### Anmerkung:

Anzumerken ist, dass in einer Beduftungsvorrichtung die beschriebene schräge Anordnung der Einlassund/oder Auslassöffnungen auch unabhängig von der in Anspruch 1 beanspruchten Ausgestaltung mit Schiebevorrichtung vorgesehen sein kann. Beispielsweise kann eine Beduftungsvorrichtung für ein Fahrzeug mit einem Gehäuse und einem im Gehäuse angeordneten Duftstoffbehälter vorgesehen sein, wobei das Gehäuse umfasst
- eine Haltevorrichtung zum Anbringen der Beduftungsvorrichtung an einer Lüftung im Innenraum eines Fahrzeuges,
- eine Einlassseite, an welcher eine Einlassöffnung zum Einströmen von Luft in das Gehäuse angeordnet ist,
- eine, insbesondere der Einlassseite gegenüberliegenden, Auslassseite, an welcher eine Auslassöffnung zum Ausströmen von Luft aus dem Gehäuse angeordnet ist,
wobei die Auslassöffnung (5) ein länglicher Schlitz ist und der längliche Schlitz schräg zur Längsseite (61) der Auslassseite (6) angeordnet ist, insbesondere auch schräg zur Kurzseite (62) der Auslassseite (6) angeordnet ist.

Insbesondere ist der längliche Schlitz in einem Winkel von mindestens 10°, insbesondere mindestens 20°, insbesondere mindestens 30°, zur Längsseite (61) der Auslassseite (6) und/oder zur Kurzseite (61) der Auslassseite (6) angeordnet.

Insbesondere weist die Beduftungsvorrichtung mindestens drei längliche Schlitze auf.

### Kurze Beschreibung der Zeichnungen

Weitere Ausgestaltungen, Vorteile und Anwendungen der Erfindung ergeben sich aus den abhängigen Ansprüchen und aus der nun folgenden Beschreibung anhand der Figuren. Dabei zeigen:
Fig. 1 eine Beduftungsvorrichtung mit Sicht auf die Vorderseite und einem nicht eingesetzten Duftstoffbehälter;
Fig. 2 die Beduftungsvorrichtung mit Sicht auf die Rückseite, wobei zwei Gummikappen mit der Beduftungsvorrichtung gekoppelt sind;
Fig. 3 die Beduftungsvorrichtung mit Sicht auf die Rückseite, wobei ein Spreizelement mit der Beduftungsvorrichtung gekoppelt ist;
Fig. 4 eine Explosionszeichnung der Beduftungsvorrichtung ohne Duftstoffbehälter; und
Fig. 5a bis 5f eine Gummikappe in verschiedenen Ansichten.

### Weg zur Ausführung der Erfindung

### Übersicht

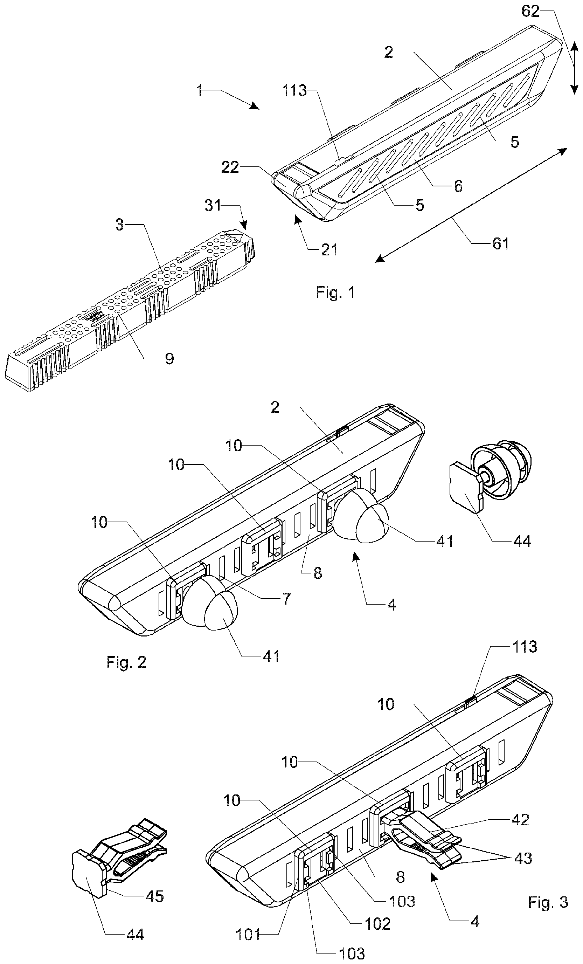

Die Figuren 1 bis 4 zeigen eine erfindungsgemässe Beduftungsvorrichtung 1 für ein Fahrzeug mit einem Gehäuse 2 und einem im Gehäuse 2 angeordneten Duftstoffbehälter 3. Der Duftstoffbehälter 3 ist auswechselbar. Er kann dem Gehäuse 2 entnommen und ein neuer Duftstoffbehälter 3 kann in das Gehäuse 2 eingesetzt werden. Die Beduftungsvorrichtung 1 weist Haltevorrichtungen 4 auf, mit welchen die Beduftungsvorrichtung 1 an einer Lüftung im Innenraum des Fahrzeuges montiert werden kann.

Die Fig. 1 zeigt die Beduftungsvorrichtung 1 mit Blick auf die Vorderseite. An der Vorderseite der Beduftungsvorrichtung 1 sind Auslassöffnungen 5 angeordnet. Die Vorderseite wird deshalb auch als Auslassseite 6 bezeichnet. Zusätzlich können auch Auslassöffnungen an einer oder an beiden Seitenwänden des Gehäuses 2 und/oder an dessen Oberseite und/oder an dessen Unterseite vorgesehen sein.

Die Fig. 2 und 3 zeigen die Beduftungsvorrichtung 1 mit Blick auf die Rückseite. An der Rückseite der Beduftungsvorrichtung 1 sind Einlassöffnungen 7 angeordnet. Die Rückseite wird deshalb auch als Einlassseite 8 bezeichnet.

Ist die Beduftungsvorrichtung 1 an der Lüftung im Innenraum des Fahrzeuges montiert, ist die Einlassseite 8 zur Lüftung gerichtet und die Auslassseite 6 ist von der Lüftung weggerichtet. Luft strömt aus der Lüftung durch die Einlassöffnungen 7, in das Gehäuse 2 und in den im Gehäuse 2 angeordneten Duftstoffbehälter 3. Die Luft kommt mit dem Duftstoff im Duftstoffbehälter 3 in Kontakt, wird mit Duftmolekülen angereichert und verlässt durch die Auslassöffnungen 5 das Gehäuse 2 der Beduftungsvorrichtung 1. Die Luft gelangt in den Innenraum des Fahrzeuges und beduftet den Innenraum.

### Duftstoffbehälter

Der Duftstoffbehälter 3, wie in Fig. 2 dargestellt, ist als auswechselbarer Duftstift vorgesehen, wobei der Duftstift als Material einen Kompositwerkstoff aus zumindest einem Polymer, insbesondere einem Elastomer, und dem Duftstoff umfasst bzw. aus einem solchen Material besteht. Dabei ist das Polymer mit dem Duftstoff angereichert und fungiert als dessen Träger. Der Duftstift weist bevorzugt mehrere Durchlassöffnungen 9 auf, durch die die aus dem Lüftungsschlitz austretende Luft strömen kann. Durch das Vorsehen der Durchlassöffnungen 9 wird die Oberfläche vergrössert, an welcher die aus dem Lüftungsschlitz austretende Luft mit dem Duftstoff in Kontakt gerät, was eine erhöhte Anreicherung der die Beduftungsvorrichtung 1 durchströmenden Luft mit Duftstoffmolekülen zur Folge hat. Die Durchlassöffnungen 9 sind vorzugsweise auf die Strömungsrichtung der Luft aus dem Lüftungsschlitz ausgerichtet. Alternativ kann der Duftstoff auch in Form eines Granulats vorgesehen sein, das in einem Käfig enthalten ist.

Wie in Fig. 1 gezeigt, verfügt das Gehäuse 2 an einem seiner Enden über eine Gehäuseöffnung 21, welche mit einem Gehäusedeckel 22 verschliessbar ist. Für den Austausch des Duftstoffbehälters 3 öffnet der Benutzer den Gehäusedeckel 22. Dieser ist über ein Drehscharnier am Gehäuse befestigt und bleibt somit auch in geöffneter Position mit dem Gehäuse 2 verbunden.

Möchte der Benutzer den neuen Duftstoffbehälter 3 einsetzen, muss er diesen in korrekter Richtung in das Gehäuse 2 bzw. in einen Schiebebehälter 11 einführen. Denn am vorderen Ende des Duftstoffbehälters 3 ist eine Vertiefung 31 vorgesehen, in welche in eingesetzter Position ein Vorsprung des Gehäuses 2 bzw. des Schiebebehälters 11 eingreift. Greifen Vertiefung 31 und Vorsprung nicht ineinander, kann der Duftstoffbehälter 3 nicht vollständig in das Gehäuse 2 eingesetzt werden und der Gehäusedeckel 22 lässt sich nicht schliessen. Hat der Benutzer den Duftstoffbehälter 3 korrekt in das Gehäuse 2 eingesetzt, kann er den Gehäusedeckel 22 schliessen. Beim Schliessen rastet der Gehäusedeckel 22 am Gehäuse 2 ein, sodass sich der Gehäusedeckel 22 nicht ungewollt öffnet.

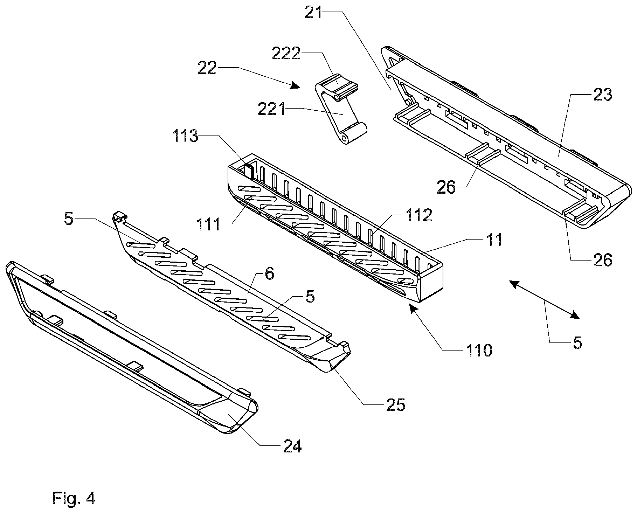

In Fig. 4 gut sichtbar ist, dass der Gehäusedeckel 22 eine gebogene Form aufweist. Der längere Abschnitt 221 des Gehäusedeckels 22 bildet das seitliche Ende des Gehäuses 2. Der kürzere Abschnitt 222 des Gehäusedeckels 22 erstreckt sich entlang eines Abschnitts der Längsseite des Gehäuses 2. Die gebogene Form des Gehäusedeckels 22 hat den Vorteil, dass die Gehäuseöffnung 21 entsprechend gross ausgestaltet ist und der Duftstoffbehälter 3 komfortabel einführbar ist.

### Ausgestaltung der Auslassseite

Wie in Fig. 1 ersichtlich, weist das Gehäuse 2 und somit auch die Auslassseite 6 eine längliche Form mit einer Längsseite 61 und einer Kurzseite 62 auf. Auf der Auslassseite 6 sind mehrere Auslassöffnungen 5 angeordnet. Sie weisen jeweils die Form eines länglichen Schlitzes auf und sind parallel und äquidistant zueinander angeordnet.

Die Auslassöffnungen 5 sind jeweils schräg zur Längsseite 61 und schräg zur Kurzseite 62 angeordnet. Mit anderen Worten sind die Auslassöffnungen 5 weder parallel zur Längsseite 61, noch parallel zur Kurzseite 62 angeordnet. Schräg bedeutet in der vorliegenden Ausführungsform, dass die als Schlitze ausgestalteten Auslassöffnungen 5 in einem Winkel von 45° zur Längsseite 61 und in einem Winkel von 45° zur Kurzseite 62 angeordnet sind. Die Auslassöffnungen 5 erstrecken sich jeweils annähernd über die gesamte Länge der Kurzseite 62.

### Haltevorrichtungen

An der Einlassseite 8 sind drei Koppelvorrichtungen 10 angeordnet. Mit jeder der drei Koppelvorrichtungen 10 kann jeweils eine Haltevorrichtung 4 gekoppelt werden. Die drei Koppelvorrichtungen 10 sind voneinander beabstandet und zwischen ihnen sind Einlassöffnungen 7 angeordnet.

In Fig. 2 ist eine erste Variante dargestellt, die Beduftungsvorrichtung 1 an die Lüftung des Fahrzeuges zu montieren. Mit den äusseren Koppelvorrichtungen 10 sind jeweils eine Haltevorrichtung 4 mit einer Gummikappe 41 gekoppelt. Die mittlere Koppelvorrichtung 10 ist freigelassen und mit keiner Haltevorrichtung 4 gekoppelt.

Die Gummikappen 41 sind aus einem elastischen Material und derart ausgestaltet, dass sie zwischen zwei Lamellen der Lüftung im Fahrzeug geklemmt werden können. Die Haltevorrichtungen 4 mit den Gummikappen 41 können somit haltend in einen Lüftungsschlitz im Fahrzeuginnenraum eingebracht werden. Das heisst, die Dimensionen der Gummikappen 41 sind in Abhängigkeit von den Dimensionen, insbesondere der Breite, des Lüftungsschlitzes derart gewählt, dass die Beduftungsvorrichtung 1 an den Lüftungsschlitz angebracht werden kann, indem die Gummikappen 41 in den Lüftungsschlitz eingeführt/eingebracht werden und dort von diesem klemmend gehalten werden. Die Gummikappen 41 sind auf die Haltevorrichtungen 4 aufsteckbar. Dadurch können die Gummikappen 41 in einfacher Weise ausgewechselt werden.

Das Gehäuse 2 ist bevorzugt länglich ausgeführt. Ist es an einem Lüftungsschlitz montiert, erstreckt es sich entlang des Lüftungsschlitzes. Beide Haltevorrichtungen 4 sind in demselben Lüftungsschlitz angeordnet. Selbstverständlich ist es auch möglich, das Gehäuse 2 quer zu den Lüftungsschlitzen anzuordnen, wobei die Haltevorrichtungen 4 dann in unterschiedliche Lüftungsschlitze eingebracht sind.

In Fig. 3 ist eine zweite Variante dargestellt, die Beduftungsvorrichtung 1 an die Lüftung des Fahrzeuges zu montieren. Die äusseren Koppelvorrichtungen 10 sind freigelassen und mit keiner Haltevorrichtung 4 gekoppelt. Lediglich an der mittleren Koppelvorrichtung 10 ist eine Haltevorrichtung 4 angeordnet, welche ein Spreizelement 42 aufweist. Das Spreizelement 42 umfasst zwei Spreizglieder 43 und kann wie die Gummikappen 41 zwischen zwei Lamellen der Lüftung des Fahrzeuges geklemmt werden.

Die Haltevorrichtung 4 mit der Gummikappe 41 und die Haltevorrichtung 4 mit dem Spreizelement 42 stellen unterschiedliche Typen von Haltevorrichtungen 4 dar, welche konstruktiv unterschiedlich ausgestaltet sind.

Alle drei Koppelvorrichtungen 10 sind identisch ausgestaltet. D.h. sowohl die Haltevorrichtungen 4 mit den Gummikappen 41 als auch die Haltevorrichtung 4 mit dem Spreizelement 42 können mit sämtlichen drei Koppelvorrichtungen gekoppelt und somit untereinander ausgetauscht werden. Anspruchsgemäss kann somit jede der Haltevorrichtungen 4 mit einer der mindestens einen Koppelvorrichtung 10 gekoppelt werden.

Wie in den Fig. 2 und 3 ersichtlich, koppeln die Koppelvorrichtungen 10 mit den Haltevorrichtungen 4 in Form einer Schnappverbindung. Die Koppelvorrichtungen 10 weisen einen quadratischen Rahmen 101 auf. Der Rahmen 101 ist auf drei Seiten geschlossen und weist an seiner Unterseite eine Rahmenöffnung 102 auf. Im Rahmen 101 sind zudem auf der linken und auf der rechten Seite jeweils zwei Lücken 103 angeordnet.

Die Haltevorrichtung 4 umfasst eine ebene, quadratische Platte 44 mit welcher je nach Typ der Haltevorrichtung 4 entweder die Gummikappen 41 oder das Spreizelement 42 verbunden sind. An der Platte 44 sind Erhebungen 45 angeordnet, welche in eingeschnappter Position in die Lücken 103 des Rahmens 101 der Koppelvorrichtung 10 eingreifen können.

Ein Benutzer kann die Haltevorrichtung 4 mit der Koppelvorrichtung 10 koppeln, indem er die Platte 44 der Haltevorrichtung 4 von unten durch die Rahmenöffnung 102 unter den Rahmen 101 schiebt. Dabei deformiert sich der Rahmen 101 elastisch, weil die Erhebungen 45 auf den Rahmen 101 drücken. Der Benutzer schiebt die Platte 44 weiter, bis die Erhebungen 45 in den Lücken 103 erscheinen. Der Rahmen 101 deformiert sich zurück, und die Platte 44 ist im Rahmen 101 eingeschnappt. Haltevorrichtung 4 und Koppelvorrichtung 10 sind miteinander gekoppelt.

Möchte ein Benutzer die Haltevorrichtung 4 von der Koppelvorrichtung 10 entkoppeln, bewegt er die Haltevorrichtung 4 nach unten, sodass die Platte 44 durch die Rahmenöffnung 102 aus dem Rahmen 101 geführt wird. Der Benutzer muss für diese Bewegung, d.h. für die Aufhebung der Schnappverbindung eine gewisse Kraft aufbringen, da sich der Rahmen 101 aufgrund der Erhebungen 45 erneut elastisch deformiert.

Wie bereits beschrieben, stehen dem Benutzer unterschiedliche Varianten zur Verfügung, um die Beduftungsvorrichtung 1 an die Lüftung im Fahrzeug zu montieren. Einerseits kann der Benutzer zur Montage die Haltevorrichtungen 4 mit den Gummikappen 41, andererseits die Haltevorrichtung 4 mit dem Spreizelement 42 verwenden. Der Benutzer verfügt somit über ein Kit, welches einerseits das Gehäuse 2 mit dem Duftstoffbehälter 3 und andererseits die unterschiedlichen Typen von Haltevorrichtungen 4 umfasst. Zusätzlich können im Kit auch unterschiedlich grosse Gummikappen 41 vorhanden sein. D.h. dem Benutzer stehen in diesem Fall insgesamt drei Varianten zur Verfügung, um die Beduftungsvorrichtung 1 an der Lüftung im Innenraum des Fahrzeuges zu montieren. Die Montage erfolgt mittels den kleineren Gummikappen 41, mittels den grösseren Gummikappen 41 oder mittels des Spreizelements 42. Der Benutzer wählt aus den drei Varianten diejenige aus, welche sich in Abhängigkeit der Ausgestaltung der Lüftung am besten eignet, um die Beduftungsvorrichtung 1 zu montieren.

### Schiebevorrichtung

Fig. 4 zeigt eine Explosionsdarstellung der Einzelteile der Beduftungsvorrichtung 1. Der Duftstoffbehälter 3 ist nicht sichtbar. Das Gehäuse 2 umfasst eine rückseitige Gehäusehülle 23, einen frontseitigen Gehäuserahmen 24, welcher eine frontseitige Gehäuseabdeckung 25 einrahmt. Die Gehäuseabdeckung 25 umfasst die bereits erwähnten Auslassöffnungen 5 an der Auslassseite 6. Ebenfalls gezeigt ist der bereits erwähnte Gehäusedeckel 22, welcher die Gehäuseöffnung 21 verschliessen kann.

Innerhalb des Gehäuses 2 ist eine Schiebevorrichtung mit einem Schiebebehälter 11 angeordnet. Der Schiebebehälter umfasst einen Boden 110, eine vordere Wand mit schrägen Auslassöffnungen 111 und ein rückseitige Wand mit vertikalen Einlassöffnungen 112. Zwischen den Wänden des Schiebebehälters 11 ist Raum vorhanden, sodass der Duftstoffbehälter 3 innerhalb des Schiebebehälters 11 angeordnet werden kann.

An der Innenseite des Gehäuses 2 sind Stege 26 vorhanden, auf welchen sich der Schiebebehälter 11 bodenseitig abstützt oder welche er mit seinen seitlichen Wänden berührt. Dadurch ist die Kontaktfläche zwischen dem Gehäuse 2 und dem Schiebebehälter 11 möglichst klein gehalten. Der Schiebebehälter 11 lässt sich innerhalb des Gehäuses 2 leicht verschieben.

Zur Bewegung des Schiebebehälters 11 ist an seiner Oberseite ein plattenförmiger Griff 113 angeordnet, welcher durch die Oberseite des Gehäuses 2 aus diesem herausragt. Der Griff 113 ragt somit weder durch die Einlassseite 8, noch durch die Auslassseite 6. Der Griff 113 ist auch in Fig. 1 sichtbar. Durch Betätigung des Griffes 113 kann der Benutzer die Schiebevorrichtung 11 relativ zum Gehäuse 2 in Längsrichtung verschieben. Der Schiebebehälter 11 wird dabei in Richtung des in Fig. 4 gezeigten Doppelpfeils 114 hin- und her bewegt.

Da der Duftstoffbehälter 3 innerhalb des Schiebebehälters 11 angeordnet ist und das Gehäuse 2 nicht berührt, bewegt sich der Duftstoffbehälter 3 in gleicherweise wie der Schiebebehälter 11 relativ zum Gehäuse 2. Dadurch reibt sich der Duftstoffbehälter 3 während der Bewegung nicht mit dem Gehäuse 2. Reibung entsteht lediglich zwischen dem Schiebebehälter 11 und den Stegen 26 des Gehäuses 2. Die Bewegung erfolgt somit kontrolliert, mit nur geringfügiger Reibung und ist unabhängig davon, ob der Duftstoffbehälter 3 vom Benutzer sauber im Schiebebehälter 11 eingesetzt wurde.

Mittels der Bewegung des Schiebebehälters 11 kann der Benutzer die Einlassöffnungen 7 und die Auslassöffnungen 5 öffnen und schliessen. Im geschlossenen Zustand verdecken die Streben des Schiebebehälters 11 die Einlassöffnungen 7 und die Auslassöffnungen 5. Luft kann die Beduftungsvorrichtung 1 nicht durchströmen. Im offenen Zustand sind die Auslassöffnungen 111 des Schiebebehälters 11 deckungsgleich mit den Auslassöffnungen 5 des Gehäuses 2 und die Einlassöffnungen 112 des Schiebebehälters 11 sind deckungsgleich mit den Einlassöffnungen 7 des Gehäuses 2. Luft kann durch die Beduftungsvorrichtung 1 strömen und mit Duftstoffmolekülen des Duftstoffbehälters 3 angereichert werden.

### Ausgestaltung der Gummikappen

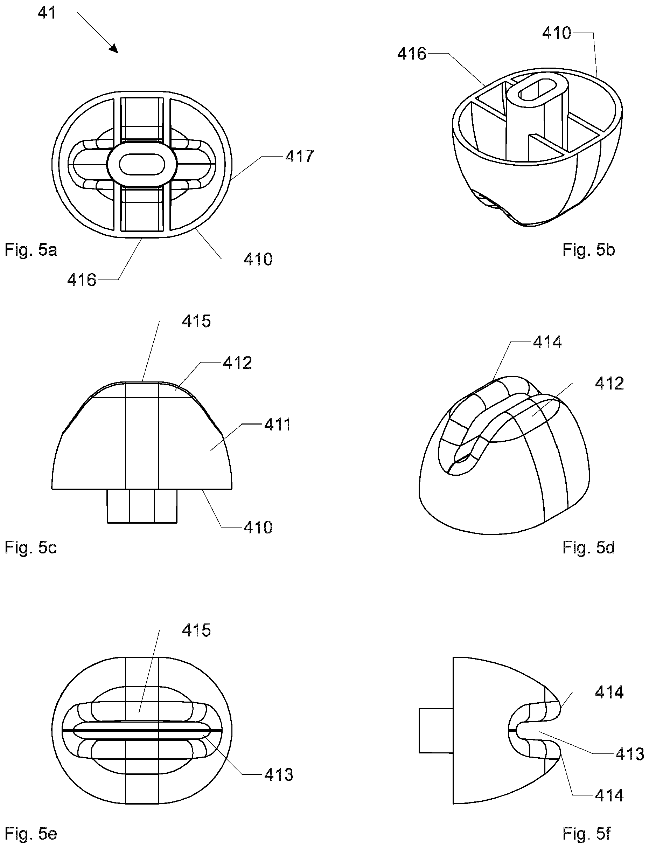

Fig. 5a bis 5f zeigen die an den Haltevorrichtungen 4 angeordneten Gummikappen 41 in detaillierter Ansicht und aus verschiedenen Perspektiven. Die Gummikappe 41 weist eine Grundfläche 410, eine Mantelfläche 411 und eine der Grundfläche 410 gegenüberliegende Oberseite 412 auf. Da die in Fig. 6 dargestellte Gummikappe 41 eine abgerundete Oberseite 412 aufweist, bildet die Oberseite 412 einen Bestandteil der Mantelfläche 411.

Bei der Grundfläche handelt es sich um diejenige Fläche der Gummikappe, welche im montierten Zustand zum Gehäuse der Beduftungsvorrichtung hingerichtet ist. Bei der Oberseite handelt es sich um diejenige Fläche der Gummikappe, welche im montierten Zustand vom Gehäuse der Beduftungsvorrichtung weggerichtet ist.

An der Oberseite 412 der Gummikappe 41 ist eine längliche Nut 413 angeordnet. Die Nut 413 trennt die Oberseite 412 in zwei parallel verlaufende Oberkanten 414. Diese Oberkanten 414 weisen jeweils einen geradlinigen Abschnitt 415 auf.

Die Grundfläche 410 der Gummikappe 41 weist einen Umfang bzw. eine Randlinie mit zwei geradlinigen Abschnitten 416 auf. Diese beiden geradlinigen Abschnitte 416 werden durch zwei Halbkreise 417 miteinander verbunden. Die Grundfläche 410 ähnelt somit einer elliptischen Form, wobei es sich aber gerade nicht um eine elliptische Form handelt, weil die Randlinie geradlinige Abschnitte 416 aufweist.

Während in der vorliegenden Anmeldung bevorzugte Ausführungen der Erfindung beschrieben sind, ist klar darauf hinzuweisen, dass die Erfindung nicht auf diese beschränkt ist und in auch anderer Weise innerhalb des Umfangs der folgenden Ansprüche ausgeführt werden kann.

## Patentansprüche

1. Beduftungsvorrichtung (1) für ein Fahrzeug mit einem Gehäuse (2) und einem im Gehäuse (2) angeordneten Duftstoffbehälter (3), wobei das Gehäuse (2) umfasst
- eine Haltevorrichtung (4) zum Anbringen der Beduftungsvorrichtung (1) an einer Lüftung im Innenraum eines Fahrzeuges,
- eine Einlassseite (8), an welcher eine Einlassöffnung (7) zum Einströmen von Luft in das Gehäuse (2) angeordnet ist,
- eine, insbesondere der Einlassseite (8) gegenüberliegende, Auslassseite (6), an welcher eine Auslassöffnung (5) zum Ausströmen von Luft aus dem Gehäuse (2) angeordnet ist, wobei
die Beduftungsvorrichtung eine Schiebevorrichtung mit einem im Gehäuse (2) angeordneten Schiebebehälter (11) aufweist, wobei der Duftstoffbehälter (3) im Schiebebehälter (11) angeordnet ist,
wobei mittels der Schiebevorrichtung die Auslassöffnung (5) und/oder die Einlassöffnung (7) mittels einer Schiebebewegung (114) zumindest teilweise verschliessbar sind, **dadurch gekennzeichnet, dass**
die Schiebevorrichtung derart ausgestaltet ist, dass sich bei der Schiebebewegung (114) der Duftstoffbehälter (3) relativ zum Gehäuse (2) verschiebt.

2. Beduftungsvorrichtung nach dem vorangehenden Anspruch, wobei der Duftstoffbehälter (3) im Schiebebehälter (11) derart angeordnet ist, dass sich der Duftstoffbehälter (3) bei der Schiebebewegung (114) nicht entlang des Gehäuses (2) reibt.

3. Beduftungsvorrichtung nach einem der vorangehenden Ansprüche, wobei das Gehäuse (2) Stege (26) aufweist, auf welchen der Schiebebehälter (11) abgestützt ist.

4. Beduftungsvorrichtung nach einem der vorangehenden Ansprüche, wobei die Schiebevorrichtung einen Griff (113), insbesondere einen plattenförmigen Griff (113), aufweist, durch dessen Betätigung die Schiebevorrichtung verschiebbar ist, wobei der Griff (113) weder durch die Auslassseite (6) noch durch die Einlassseite (8) ragt.

5. Beduftungsvorrichtung nach einem der vorangehenden Ansprüche, wobei der Duftstoffbehälter (3) eine Vertiefung (31) aufweist, in welche bei eingesetztem Duftstoffbehälter (3) ein Vorsprung des Schiebebehälters (11) ragt.

6. Beduftungsvorrichtung nach einem der vorangehenden Ansprüche, wobei die Auslassseite (6) eine längliche Form mit einer Längsseite (61) und einer Kurzseite (62) aufweist, und wobei die Auslassöffnung (5) ein länglicher Schlitz ist und der längliche Schlitz schräg zur Längsseite (61) der Auslassseite (6) angeordnet ist, insbesondere auch schräg zur Kurzseite (62) der Auslassseite (6) angeordnet ist.

7. Beduftungsvorrichtung nach Anspruch 6, wobei der längliche Schlitz in einem Winkel von mindestens 10°, insbesondere mindestens 20°, insbesondere mindestens 30°, zur Längsseite (61) der Auslassseite (6) und/oder zur Kurzseite (61) der Auslassseite (6) angeordnet ist.

8. Beduftungsvorrichtung nach einem der Ansprüche 6 oder 7, aufweisend mindestens drei längliche Schlitze.

9. Beduftungsvorrichtung nach Anspruch 8, wobei die länglichen Schlitze parallel zueinander angeordnet sind, und/oder äquidistant zueinander angeordnet sind, und/oder gleich lang sind.

10. Beduftungsvorrichtung nach einem der Ansprüche 6 bis 9, wobei der längliche Schlitz sich im Wesentlichen über die gesamte Kurzseite (62) der Auslassseite erstreckt.

11. Beduftungsvorrichtung nach einem der vorangehenden Ansprüche, wobei der Duftstoffbehälter (3) durch eine Gehäuseöffnung (21) in das Gehäuse (2) einsetzbar und diesem wieder entnehmbar ist,
insbesondere wobei der Duftstoffbehälter (3) in Form eines Duftstifts ausgestaltet ist.

12. Beduftungsvorrichtung nach Anspruch 11, wobei die Gehäuseöffnung (21) mittels eines Gehäusedeckels (22) öffen- und schliessbar ist, wobei der Gehäusedeckel (22) mittels eines Drehscharniers am Gehäuse (2) angeordnet ist,
insbesondere wobei genau eine einzige Gehäuseöffnung (21) vorhanden ist,
insbesondere wobei der Gehäusedeckel (22) in geschlossenem Zustand eingerastet ist.

13. Beduftungsvorrichtung nach Anspruch 12, wobei sich ein Teil des Gehäusedeckels (22) entlang der Längsseite (61) erstreckt.

14. Beduftungsvorrichtung nach einem der Ansprüche 12 bis 13, wobei der Gehäusedeckel (22) nur schliessbar ist, wenn der Duftstoffbehälter (3) in korrekter Position und/oder korrekt ausgerichtet im Gehäuse (2) eingesetzt ist, wobei es nur genau eine korrekte Position und/oder nur genau eine korrekte Ausrichtung gibt.

## Claims

1. Scenting device (1) for a vehicle with a housing (2) and a scent container (3) arranged in the housing (2), wherein the housing (2) comprises
- a holding device (4) for attaching the scenting device (1) to a ventilation system in the interior of a vehicle,
- an inlet side (8), on which an inlet opening (7) for the inflow of air into the housing (2) is arranged,
- an outlet side (6), in particular opposite the inlet side (8), at which an outlet opening (5) for the outflow of air from the housing (2) is arranged, wherein
the scenting device has a sliding device with a sliding container (11) arranged in the housing (2), the scent container (3) being arranged in the sliding container (11),
wherein the outlet opening (5) and/or the inlet opening (7) can be at least partially closed by means of the sliding device by means of a sliding movement (114), **characterised in that** the sliding device is adapted in such a way that the scent container (3) is displaced relative to the housing (2) during the sliding movement (114).

2. Scenting device according to the preceding claim, wherein the scent container (3) is arranged in the sliding container (11) in such a way that the scent container (3) does not rub along the housing (2) during the sliding movement (114).

3. Scenting device according to one of the preceding claims, wherein the housing (2) has bars (26) on which the sliding container (11) is supported.

4. Scenting device according to one of the preceding claims, wherein the sliding device has a handle (113), in particular a plate-shaped handle (113), by the actuation of which the sliding device is slidable, wherein the handle (113) projects neither through the outlet side (6) nor through the inlet side (8).

5. Scenting device according to one of the preceding claims, wherein the scent container (3) has a recess (31) into which a projection of the sliding container (11) projects when the scent container (3) is inserted.

6. Scenting device according to one of the preceding claims, wherein the outlet side (6) has an elongate shape with a long side (61) and a short side (62), and wherein the outlet opening (5) is an elongate slit and the elongate slit is arranged obliquely to the long side (61) of the outlet side (6), in particular is also arranged obliquely to the short side (62) of the outlet side (6).

7. Scenting device according to claim 6,
wherein the elongate slit is arranged at an angle of at least 10°, in particular at least 20°, in particular at least 30°, to the long side (61) of the outlet side (6) and/or to the short side (61) of the outlet side (6).

8. Scenting device according to one of claims 6 or 7, comprising at least three elongated slits.

9. Scenting device according to claim 8,
wherein the elongated slits are arranged parallel to each other, and/or are arranged equidistant to each other, and/or are of equal length.

10. Scenting device according to any one of claims 6 to 9, wherein the elongate slit extends substantially over the entire short side (62) of the outlet side.

11. Scenting device according to one of the preceding claims, wherein the scent container (3) can be inserted into the housing (2) through a housing opening (21) and can be removed therefrom again,
in particular wherein the scent container (3) is designed in the form of a scent stick.

12. Scenting device according to claim 11, wherein the housing opening (21) can be opened and closed by means of a housing cover (22), wherein the housing cover (22) is arranged on the housing (2) by means of a rotary hinge,
in particular wherein exactly one single housing opening (21) is present,
in particular wherein the housing cover (22) is latched in the closed state.

13. Scenting device according to claim 12, wherein a part of the housing cover (22) extends along the longitudinal side (61).

14. Scenting device according to one of claims 12 to 13, wherein the housing cover (22) can only be closed when the scent container (3) is inserted in the housing (2) in the correct position and/or correctly aligned, wherein there is only exactly one correct position and/or correct alignment of the housing cover (22).

## Revendications

1. Dispositif de diffusion de parfum (1) pour un véhicule, comprenant un boîtier (2) et un réservoir de parfum (3) disposé dans le boîtier (2), le boîtier (2) comprenant
- un dispositif de support (4) pour fixer le dispositif de diffusion de parfum (1) à une ventilation dans l'habitacle d'un véhicule,
- un côté d'entrée (8) sur lequel est disposée une ouverture d'entrée (7) pour l'afflux d'air dans le boîtier (2),
- un côté de sortie (6), en particulier opposé au côté d'entrée (8), sur lequel est disposée une ouverture de sortie (5) pour l'écoulement d'air hors du boîtier (2), dans lequel
le dispositif de diffusion de parfum présente un dispositif coulissant avec un récipient coulissant (11) disposé dans le boîtier (2), le récipient de parfum (3) étant disposé dans le récipient coulissant (11),
l'ouverture de sortie (5) et/ou l'ouverture d'entrée (7) pouvant être fermées au moins partiellement au moyen du dispositif coulissant par un mouvement de coulissement (114), **caractérisé en ce que** le dispositif coulissant est conçu de telle sorte que, lors du mouvement de coulissement (114), le récipient de parfum (3) se déplace par rapport au boîtier (2).

2. Dispositif de diffusion de parfum selon la revendication précédente, dans lequel le réservoir de parfum (3) est disposé dans le réservoir coulissant (11) de telle sorte que le réservoir de parfum (3) ne frotte pas le long du boîtier (2) lors du mouvement de coulissement (114).

3. Dispositif de diffusion de parfum selon l'une des revendications précédentes, dans lequel le boîtier (2) comporte des entretoises (26) sur lesquelles le réservoir coulissant (11) est en appui.

4. Dispositif de diffusion de parfum selon l'une des revendications précédentes, dans lequel le dispositif coulissant comporte une poignée (113), notamment une poignée (113) en forme de plaque, dont l'actionnement permet de faire coulisser le dispositif coulissant, la poignée (113) ne faisant saillie ni à travers le côté de sortie (6) ni à travers le côté d'entrée (8).

5. Dispositif de diffusion de parfum selon l'une des revendications précédentes, dans lequel le réservoir de parfum (3) présente une cavité (31) dans laquelle une saillie du réservoir coulissant (11) fait saillie lorsque le réservoir de parfum (3) est inséré.

6. Dispositif de diffusion de parfum selon l'une des revendications précédentes, dans lequel le côté de sortie (6) présente une forme allongée avec un côté longitudinal (61) et un côté court (62), et dans lequel l'ouverture de sortie (5) est une fente allongée et la fente allongée est disposée en biais par rapport au côté longitudinal (61) du côté de sortie (6), en particulier est également disposée en biais par rapport au côté court (62) du côté de sortie (6).

7. Dispositif de diffusion de parfum selon la revendication 6, dans lequel la fente allongée est disposée selon un angle d'au moins 10°, en particulier d'au moins 20°, notamment d'au moins 30°, par rapport au côté longitudinal (61) du côté de sortie (6) et/ou par rapport au côté court (61) du côté de sortie (6).

8. Dispositif de diffusion de parfum selon l'une des revendications 6 ou 7, comprenant au moins trois fentes allongées.

9. Dispositif de diffusion de parfum selon la revendication 8, dans lequel les fentes allongées sont disposées parallèlement les unes aux autres, et/ou sont équidistantes les unes des autres, et/ou sont de même longueur.

10. Dispositif de diffusion de parfum selon l'une des revendications 6 à 9, dans lequel la fente allongée s'étend sensiblement sur tout le petit côté (62) du côté de la sortie.

11. Dispositif de diffusion de parfum selon l'une des revendications précédentes, dans lequel le réservoir de parfum (3) peut être inséré dans le boîtier (2) et retiré de celui-ci par une ouverture (21) du boîtier,
en particulier dans lequel le réservoir de parfum (3) est conçu sous la forme d'un bâton de parfum.

12. Dispositif de diffusion de parfum selon la revendication 11, dans lequel l'ouverture de boîtier (21) peut être ouverte et fermée au moyen d'un couvercle de boîtier (22), le couvercle de boîtier (22) étant disposé sur le boîtier (2) au moyen d'une charnière rotative,
en particulier dans lequel il existe exactement une seule ouverture de boîtier (21),
en particulier, le couvercle (22) du boîtier étant encliqueté à l'état fermé.

13. Dispositif de diffusion de parfum selon la revendication 12, dans lequel une partie du couvercle de boîtier (22) s'étend le long du côté longitudinal (61).

14. Dispositif de diffusion de parfum selon l'une des revendications 12 à 13, dans lequel le couvercle de boîtier (22) ne peut être fermé que lorsque le réservoir de parfum (3) est inséré dans le boîtier (2) dans la position correcte et/ou dans l'orientation correcte, étant entendu qu'il n'existe qu'une seule position correcte et/ou qu'une seule orientation correcte.
